# EUROPÄISCHE PATENTSCHRIFT

(11) **EP 0 716 654 B1**
(45) Veröffentlichungstag und Bekanntmachung des Hinweises auf die Patenterteilung: **17.01.2001**
(21) Anmeldenummer: 94927503.6
(22) Anmeldetag: 25.08.1994
(51) Int. Cl.: C07D 239/60, C07D 405/12, C07D 401/12, C07D 409/12, A01N 43/54, A01N 25/32

(54) **SUBSTITUIERTE MILCHSÄUREDERIVATE MIT EINEM AZIDO-REST IN BETA-POSITION, DEREN HERSTELLUNG UND DEREN VERWENDUNG ALS HERBIZIDE UND ANTIDOTE**
SUBSTITUTED LACTIC-ACID DERIVATIVES WITH AN AZIDO-GROUP IN THE BETA POSITION, THEIR PREPARATION AND THEIR USE AS HERBICIDES AND ANTIDOTES
DERIVES D'ACIDE LACTIQUE SUBSTITUES COMPORTANT UN RESTE AZIDO EN POSITION BETA, LEUR PREPARATION ET LEUR UTILISATION COMME HERBICIDES ET COMME ANTIDOTES

(30) Priorität: 04.09.1993 DE 4329911
(43) Veröffentlichungstag der Anmeldung: 19.06.1996
(73) Patentinhaber: BASF AKTIENGESELLSCHAFT, 67056 Ludwigshafen (DE)
(72) Erfinder: BAUMANN, Ernst, D-67373 Dudenhofen (DE); RHEINHEIMER, Joachim, D-67063 Ludwigshafen (DE); VOGELBACHER, Uwe, Josef, D-67071 Ludwigshafen (DE); GERBER, Matthias, D-67117 Limburgerhof (DE); RADEMACHER, Wilhelm, D-67117 Limburgerhof (DE); WALTER, Helmut, D-67283 Obrigheim (DE); WESTPHALEN, Karl-Otto, D-67346 Speyer (DE)
(86) Internationale Anmeldenummer: EP9402825
(87) Internationale Veröffentlichungsnummer: WO9507266

(56) Entgegenhaltungen:
- EP-A- 0 094 348
- EP-A- 0 112 280
- EP-A- 0 122 231
- EP-A- 0 347 811
- EP-A- 0 411 706
- EP-A- 0 514 551
- EP-A- 0 517 215
- EP-A- 0 537 463
- EP-A- 0 548 710
- WO-A-94/22310
- US-A- 4 118 567
- CHEMICAL ABSTRACTS, vol. 118, no. 23, 7. Juni 1993, Columbus, Ohio, US; abstract no. 234083m, GO A. ET AL. 'Preparation of 3-amino-2-(2-pyrimidinylthio)propanoic acid derivatives as herbicides' Seite 1007 ;Spalte 1 ; & JP,A,04 334 372 (MITSUBISHI PETROCHEMICAL CO., LTD.) 20. November 1992 in der Anmeldung erwähnt

## Beschreibung

Die vorliegende Erfindung betrifft substituierte Milchsäurederivate mit einem Azido-Rest in β-Position der allgemeinen Formel I 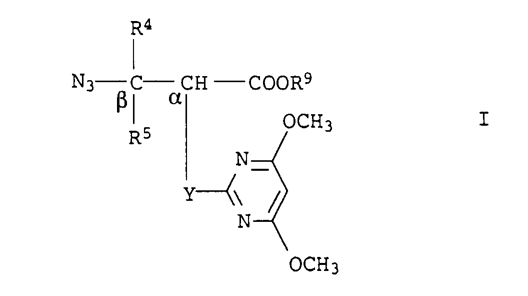 in der die Variablen folgende Bedeutung haben:
- R⁹: Wasserstoff, ein Alkalimetallkation, das Äquivalent eines Erdalkalimetallkations, das Ammoniumkation oder ein organisches Ammoniumion;
C₃-C₈-Cycloalkyl, welches ein bis drei C₁-C₄-Alkylgruppen tragen kann; C₁-C₈-Alkyl, welches Halogen, C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl oder durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy tragen kann;
C₃-C₆-Alkenyl oder C₃-C₆-Alkinyl, welche ein bis fünf Halogenatome tragen können;
Phenyl, welches Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio tragen kann;
- R⁹: ferner eine Gruppe 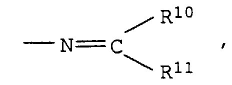 worin R¹⁰ und R¹¹, die gleich oder verschieden sein können, bedeuten:
C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio oder einen Phenylrest tragen können, welcher Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio tragen kann; oder
Phenyl, welches Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio tragen kann;
oder R¹⁰ und R¹¹ bilden gemeinsam eine C₃-C₁₂-Alkylenkette, welche ein bis drei C₁-C₄-Alkylgruppen tragen kann;
- R⁴: C₃-C₈-Cycloalkyl, C₃-C₈-Cycloalkenyl, wobei diese Reste durch Halogen, C₁-C₄-Alkyl, C₁-C₄-Alkoxy, C₁-C₄-Alkylthio, Cyano, C₁-C₄-Alkylcarbonyl, C₁-C₄-Alkoxycarbonyl oder Phenyl substituiert sein können;
C₁-C₈-Alkyl, welches Halogen, C₁-C₄-Alkoxy, C₁-C₄-Alkylthio, Cyano, Hydroxy, C₁-C₄-Alkylcarbonyl, C₁-C₄-Alkoxycarbonyl, Phenyl, Phenoxy und/oder Phenylcarbonyl tragen kann;
C₃-C₆-Alkenyl oder C₃-C₆-Alkinyl, welche Halogen, C₁-C₄-Alkyl, C₁-C₄-Alkoxy, C₁-C₄-Alkylthio, Cyano, C₁-C₄-Alkylcarbonyl, C₁-C₄-Alkoxycarbonyl oder Phenyl tragen können;
Phenyl oder Naphthyl, welche Halogen, Nitro, Cyano, Hydroxy, Mercapto, C₁-C₄-Alkyl, C₁-C₄-Halogenalkyl, C₁-C₄-Alkoxy, C₁-C₄-Halogenalkoxy, C₁-C₄-Alkylthio, Amino, C₁-C₄-Alkylamino, C₁-C₄-Dialkylamino, C₁-C₄-Alkylcarbonyl oder C₁-C₄-Alkoxycarbonyl tragen können; oder
ein fünf- oder sechsgliedriger Heteroaromat, enthaltend ein bis drei Stickstoffatome und/oder ein Schwefel- oder Sauerstoffatom;
oder R⁴ und R⁵ bilden zusammen mit dem benachbarten Kohlenstoffatom einen 3- bis 8-gliedrigen Ring;
- R⁵: Wasserstoff, C₁-C₄-Alkyl, C₃-C₆-Alkenyl, C₃-C₆-Alkinyl, C₁-C₄-Alkylcarbonyl, C₁-C₄-Alkoxycarbonyl, C₁-C₄-Halogenalkyl, C₃-C₈-Cycloalkyl, C₁-C₄-Alkoxyalkyl, C₁-C₄-Alkylthioalkyl oder Phenyl,
oder R⁵ ist mit R⁴ wie oben angegeben zu einem Ring verknüpft;
- Y: Schwefel oder Sauerstoff.

In der Literatur, z.B. EP-A 347 811, EP-A 400 741, EP-A 409 368, EP-A 411 706, EP-A 481 512, EPA 514 551, EP-A 517 215, EP-A 541 041, JP 043 34372-A und EP-A 548 710 sind ähnliche Verbindungen als herbizid wirksam beschrieben. Die biologische Wirkung und Selektivität dieser Verbindungen ist jedoch nicht immer befriedigend.

Der Erfindung lag deshalb die Aufgabe zugrunde, weitere substituierte 2-Pyrimidinyloxi(-thio)-propionsäurederivate bereitzustellen, die als selektive Herbizide mit bioregulatorischer Wirkung eingesetzt werden können.

Es wurde nun gefunden, daß die eingangs definierten 2-Pyrimidinyloxi(-thio)-propionsäurederivate I ausgezeichnete herbizide und pflanzenwachstumsregulierende Eigenschaften haben. Darüber hinaus wurde gefunden, daß die 2-Pyrimidinyloxi(-thio)-propionsäurederivate I eine gute antagonistische Wirkung gegenüber Herbiziden vom Cyclohexenontyp XIII aufweisen.

Gegenstand der Erfindung sind daher auch herbizide Mittel, enthaltend ein Milchsäurederivat der Formel I und mindestens einen herbiziden Wirkstoff aus der Gruppe der Cyclohexenon-Derivate der allgemeinen Formel XIII 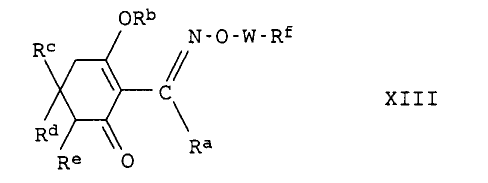 in der die Substituenten folgende Bedeutung haben:
- R^{a}: eine C₁-C₆-Alkylgruppe;
- R^{b}: Wasserstoff, das Äquivalent eines landwirtschaftlich brauchbaren Kations, eine C₂-C₈-Alkylcarbonylgruppe, eine C₁-C₁₀-Alkylsulfonylgruppe, eine C₁-C₁₀-Alkylphosphonylgruppe oder die Benzoyl-, Benzolsulfonyl- oder Benzolphosphonylgruppe, wobei die drei letztgenannten Gruppen noch je 1 bis 5 Halogenatome tragen können;
- R^{c}: Wasserstoff oder ein C-organischer Rest
- R^{d}: Wasserstoff, die Hydroxylgruppe oder, wenn R^{c} für eine C₁-C₆-Alkylgruppe steht, eine C₁-C₆-Alkylgruppe;
- R^{e}: Wasserstoff, Halogen, die Cyanogruppe, eine C₁-C₄-Alkoxycarbonylgruppe oder eine C₁-C₄-Alkylketoximgruppe;
- W: eine C₁-C₆-Alkylen-, C₃-C₆-Alkenylen- oder C₃-C₆-Alkinylenkette, die jeweils noch ein bis drei Reste tragen können, ausgewählt aus einer Gruppe bestehend aus ein bis drei C₁-C₃-Alkylsubstituenten, ein bis drei Halogenatomen und einem Methylensubstituenten;
eine C₃-C₆-Alkylen- oder C₄-C₆-Alkenylenkette, die beide noch eine bis drei C₁-C₃-Alkylreste tragen können, wobei jeweils eine Methylengruppe der Ketten durch ein Sauerstoff- oder Schwefelatom, eine Sulfoxid- oder Sulfongruppe oder eine Gruppe -N(Rⁱ)- substituiert sein kann, wobei R¹ für Wasserstoff, C₁-C₄-Alkyl, C₃-C₆-Alkenyl oder C₃-C₆-Alkinyl steht;
- R^{f}: Wasserstoff; eine optionell substituierte Vinyl- oder Ethinylgruppe, gegebenenfalls substituiertes Phenyl oder ein gegebenenfalls substituierter 5- bis 6-gliedriger heteroaromatischer Rest.

Die Herstellung der erfindungsgemäßen Verbindungen ist auf verschiedenen Wegen möglich, die im nachfolgenden beschrieben werden.

### Herstellungsweg A

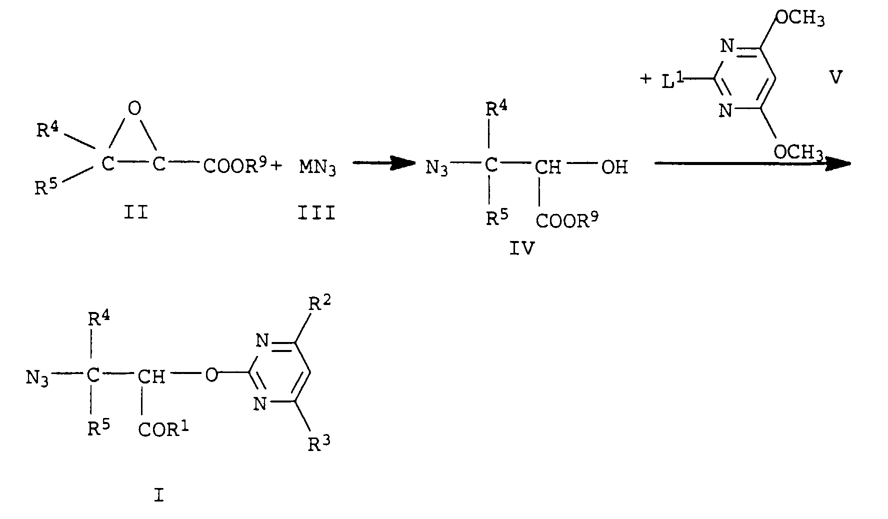

Durch Umsetzung von Epoxiden der allgemeinen Formel II, die bekannt sind oder nach allgemeinem Fachwissen, ausgehend von bekannten Vorprodukten, (vgl. z.B. J. March, Advanced Organic Chemistry, 2nd Edition, 1983, S. 750 und 862) hergestellt werden können, mit einem geeigneten Azid MN₃ III, in dem M Wasserstoff, ein Alkalimetallkation (z.B. Na, K), das Äquivalent eines Erdalkalimetallkations (z.B. Mg) oder einen Silylrest SiR₃ bedeutet, wobei die Reste R gleich oder verschieden sein können und z.B. niedermolekulares Alkyl oder Phenyl bedeuten, erhält man die Azidoalkohole der allgemeinen Formel IV, in denen R⁹, R⁴ und R⁵ die in Anspruch 1 genannte Bedeutung haben.

Die Reaktion kann auch in Gegenwart eines Verdünnungsmittels erfolgen. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Die Reaktion wird dabei bevorzugt in einem Temperaturbereich zwischen 0°C und dem Siedepunkt des Lösungsmittels bzw. Lösungsmittelgemisches durchgeführt.

Die Gegenwart eines Reaktionskatalysators kann von Vorteil sein. Als Katalysatoren kommen dabei organische Säuren und anorganische Säuren sowie Lewissäuren in Frage. Beispiele hierfür sind unter anderem Schwefelsäure, Salzsäure, Trifluoressigsäure, Bortrifluorid-Etherat, Titan(IV)-Halogenide und Trimethylsilyltriflat.

Die erfindungsgemäßen Verbindungen, in denen Y Sauerstoff bedeutet und die restlichen Substituenten die unter der allgemeinen Formel I genannte Bedeutung haben, können beispielsweise derart hergestellt werden, daß man die Azidoalkohole der allgemeinen Formel IV mit Verbindungen der allgemeinen Formel V, in der L¹ für eine übliche nucleofuge Abgangsgruppe wie Halogen, z.B. Chlor oder Brom, Alkylsulfonyl, beispielsweise C₁-C₄-Alkylsulfonyl wie Methylsulfonyl oder Arylsulfonyl, z.B. Phenylsulfonyl, steht, zur Reaktion bringt.

Die Reaktion findet bevorzugt in einem der oben genannten Verdünnungsmittel unter Zusatz einer geeigneten Base, d. h. einer Base, die die Verbindung IV zu deprotonieren vermag, in einem Temperaturbereich von Raumtemperatur bis zum Siedepunkt des Lösungsmittels statt.

Als Base kann ein Alkali- oder Erdalkalimetallhydrid wie Natriumhydrid, Kaliumhydrid oder Calciumhydrid, ein Carbonat, z. B. Alkalimetallcarbonat wie Natrium- oder Kaliumcarbonat, ein Alkalimetallhydroxid wie Natrium- oder Kaliumhydroxid, eine metallorganische Verbindung wie Butyllithium oder ein Alkaliamid wie Lithiumdiisopropylamid dienen.

### Herstellungsweg B

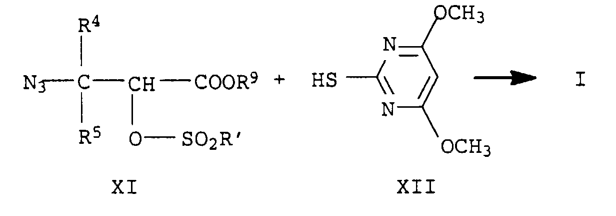

Die erfindungsgemäßen Verbindungen, in denen Y Schwefel bedeutet und die restlichen Substituenten die unter der Formel I angegebene Bedeutung haben, können beispielsweise derart hergestellt werden, daß man Milchsäurederivate der allgemeinen Formel XI, die in bekannter Weise zum Beispiel aus Verbindungen der allgemeinen Formel IV erhältlich sind und in der R' niedermolekulares Alkyl oder Halogenalkyl, z.B. mit bis zu 6 Kohlenstoffatomen oder gegebenenfalls substituiertes Phenyl bedeutet und die übrigen Substituenten die oben angegebene Bedeutung haben, mit Verbindungen der allgemeinen Formel XII, die bekannt sind oder die mit dem allgemeinen Fachwissen, ausgehend von bekannten Vorprodukten, hergestellt werden können, zur Reaktion bringt.

Die Reaktion findet bevorzugt in einem der oben genannten inerten Verdünnungsmittel unter Zusatz einer geeigneten Base, d. h. einer Base, die das Zwischenprodukt XII zu deprotonieren vermag, in einem Temperaturbereich von Raumtemperatur bis zum Siedepunkt des Lösungsmittels statt.

Als Base können neben den oben genannten auch organischen Basen wie Triethylamin, Pyridin, Imidazol oder Diazabicycloundecan dienen.

### Herstellungsweg C

Verbindungen der Formel I können auch dadurch hergestellt werden, daß man von den entsprechenden Carbonsäuren, d. h. Verbindungen der Formel I, in denen R⁹ Wasserstoff bedeutet, ausgeht und diese zunächst auf übliche Weise in eine aktivierte Form wie ein Halogenid, ein Anhydrid oder Imidazolid überführt und dieses dann mit einer entsprechenden Hydroxylverbindung HOR⁹ umsetzt. Diese Umsetzung läßt sich in den üblichen Lösungsmitteln durchführen und wird vorteilhaft in Gegenwart einer Base, wobei die oben genannten in Betracht kommen, vorgenommen. Diese beiden Schritte lassen sich beispielsweise auch dadurch vereinfachen, daß man die Carbonsäure in Gegenwart eines wasserabspaltenden Mittels wie eines Carbodiimids auf die Hydroxylverbindung einwirken läßt.

### Herstellungsweg D

Außerdem können Verbindungen der Formel I auch dadurch hergestellt werden, daß man von den Salzen der entsprechenden Carbonsäuren ausgeht, d. h. von Verbindungen der Formel I, in denen R⁹ für M steht, wobei M ein Alkalimetallkation oder das Äquivalent eines Erdalkalimetallkations sein kann. Diese Salze lassen sich mit vielen Verbindungen der Formel R⁹-A zur Reaktion bringen, wobei A eine übliche nucleofuge Abgangsgruppe bedeutet, beispielsweise Halogen wie Chlor, Brom, Jod oder gegebenenfalls durch Halogen, Alkyl oder Halogenalkyl substituiertes Aryl- oder Alkylsulfonyl wie z. B. Toluolsulfonyl und Methylsulfonyl oder eine andere äquivalente Abgangsgruppe. Verbindungen der Formel R⁹-A mit einem reaktionsfähigen Substituenten A sind bekannt oder nach allgemeinem Fachwissen leicht zugänglich. Diese Umsetzung läßt sich in den üblichen Lösungsmitteln durchführen und wird vorteilhaft in Gegenwart einer Base, wobei die oben genannten in Betracht kommen, vorgenommen.

Im Hinblick auf die biologische Wirkung sind Milchsäurederivate der Formel I bevorzugt, in der die Substituenten folgende Bedeutung haben:
- R⁹: i) Wasserstoff, das Kation eines Alkalimetalls oder das Kation eines Erdalkalimetalls wie Lithium, Natrium, Kalium, Calcium, Magnesium und Barium oder ein umweltverträgliches organisches Ammoniumion wie tert.-C₁-C₄-Alkylammonium oder Ammonium [NH₄⁺];
ii) C₃-C₈-Cycloalkyl wie Cyclopropyl, Cyclobutyl, Cyclopentyl, Cyclohexyl, Cycloheptyl und Cyclooctyl, welches z.B. ein bis drei C₁-C₄-Alkylgruppen tragen kann;
iii) C₁-C₈-Alkyl wie insbesondere Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, welches ein bis fünf Halogenatome, insbesondere Fluor oder Chlor und/oder einen der folgenden Reste tragen kann:
   C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl, ein- oder mehrfach durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy, wie insbesondere oben genannt;
iv) eine C₃-C₆-Alkenyl- oder eine C₃-C₆-Alkinylgruppe wie C₃-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Ethyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl,1,1,2-Triethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl und 3-Methyl-2-pentenyl;
   C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-Methyl-2-propinyl, vorzugsweise 2-Propinyl, 2-Butinyl, 1-Methyl-2-propinyl und 1-Methyl-2-butinyl, insbesondere 2-Propinyl;
   wobei diese Gruppen ihrerseits ein bis fünf Halogenatome tragen können;
v) R⁹ ferner ein Phenylrest, welcher ein bis fünf Halogenatome und/oder ein bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio, wie insbesondere oben genannt;
vi) R⁹ ferner eine Gruppe - 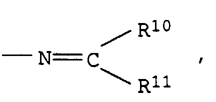 worin R¹⁰ und R¹¹, die gleich oder verschieden sein können, bedeuten: C₁-C₈-Alkyl, C₃-C₆-Alkenyl, C₃-C₆-Alkinyl, C₃-C₈-Cycloalkyl, wobei diese Reste eine C₁-C₄-Alkoxy, C₁-C₄-Alkylthio und/oder einen gegebenenfalls substituierten Phenylrest, wie insbesondere vorstehend genannt, tragen können;
   Phenyl, das durch einen oder mehrere der folgenden Reste substituiert sein kann: Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio, wobei diese Reste insbesondere den oben für R¹ genannten entsprechen;
   oder R¹⁰ und R¹¹ bilden gemeinsam eine C₃-C₁₂-Alkylenkette, welche ein bis drei C₁-C₄-Alkylgruppen tragen;
- R⁴: C₁-C₈-Alkyl wie bei R⁹ im einzelnen genannt, welches ein bis fünf Halogenatome wie Fluor, Chlor, Brom, Jod, insbesondere Fluor und Chlor und/oder einen der folgenden Reste tragen kann: C₁-C₄-Alkoxy, C₁-C₄-Alkylthio, Cyano, Hydroxy, C₁-C₄-Alkylcarbonyl, C₁-C₄-Alkoxycarbonyl, Phenyl, Phenoxy, Phenylcarbonyl wie im allgemeinen und besonderen bei R¹ genannt;
C₃-C₈-Cycloalkyl oder C₃-C₈-Cycloalkenyl, wie Cyclopropyl, Cyclobutyl, Cyclopentyl, Cyclohexyl, Cycloheptyl, Cyclooctyl, wobei die Cycloalkyl- bzw. Cycloalkenylreste substituiert sein können durch ein bis fünf Halogenatome wie vorstehend genannt, insbesondere Fluor oder Chlor und/oder einen der folgenden Reste: C₁-C₄-Alkyl, C₁-C₄-Alkoxy, C₁-C₄-Alkylthio, Cyano, C₁-C₄-Alkylcarbonyl, C₁-C₄-Alkoxycarbonyl oder Phenyl, wie im allgemeinen und besonderen oben genannt;
C₃-C₆-Alkenyl oder C₃-C₆-Alkinyl wie bei R⁹ genannt, welche ein bis fünf Halogenatome wie vorstehend genannt, insbesondere Fluor oder Chlor und/oder einen der folgenden Reste tragen können:
C₁-C₄-Alkyl, C₁-C₄-Alkoxy, C₁-C₄-Alkylthio, Cyano, C₁-C₄-Alkylcarbonyl, C₁-C₄-Alkoxycarbonyl oder Phenyl, wie im allgemeinen und besonderen oben genannt;
R⁴ ferner ein 5- oder 6-gliedriges Heteroaryl wie Furyl, Thienyl, Pyrryl, Pyrazolyl, Imidazolyl, Triazolyl, Isoxazolyl, Oxazolyl, Isothiazolyl, Thiazolyl, Thiadiazolyl, Pyridyl, Pyrimidinyl, Pyrazinyl, Pyridazinyl, Triazinyl, beispielsweise 2-Furanyl, 3-Furanyl, 2-Thienyl, 3-Thienyl, 3-Isoxazolyl, 4-Isoxazolyl, 5-Isoxazolyl, 3-Isothiazolyl, 4-Isothiazolyl, 5-Isothiazolyl, 2-Oxazolyl, 4-Oxazolyl, 5-Oxazolyl, 2-Thiazolyl, 5-Thiazolyl, 2-Imidazolyl, 4-Imidazolyl, 5-Imidazolyl, 2-Pyrrolyl, 3-Pyrrolyl, 4-Pyrrolyl, 3-Pyrazolyl, 4-Pyrazolyl, 5-Pyrazolyl, 2-Pyridyl, 3-Pyridyl, 4-Pyridyl, Oxa-2,4-diazolyl, Oxa-3,4-diazolyl, Thia-2,4-diazolyl, Thia-3,4-diazolyl, und Triazolyl, wobei die Heteroaromaten ein bis fünf Halogenatome wie vorstehend genannt, insbesondere Fluor oder Chlor und/oder einen der folgenden Reste tragen können: Nitro, Cyano, Hydroxy, Mercapto, Amino, C₁-C₄-Halogenalkyl, C₁-C₄-Halogenalkoxy, C₁-C₄-Alkylamino, C₁-C₄-Alkyl, C₁-C₄-Alkoxy, C₁-C₄-Alkylthio, Di-C₁-C₄-alkylamino, C₁-C₄-Alkylcarbonyl, C₁-C₄-Alkoxycarbonyl oder Phenyl, wie im allgemeinen und besonderen oben genannt;
R⁴ ferner Phenyl oder Naphthyl, die durch einen oder mehrere der folgenden Reste substituiert sein können: Halogen, Nitro, Cyano, Hydroxy, Mercapto, C₁-C₄-Alkyl, C₁-C₄-Halogenalkyl, C₁-C₄-Alkoxy, C₁-C₄-Halogenalkoxy, C₁-C₄-Alkylthio, Amino, C₁-C₄-Alkylamino, C₁-C₄-Dialkylamino, C₁-C₄-Alkylcarbonyl oder C₁-C₄-Alkoxycarbonyl, insbesondere wie vorstehend genannt, sowie 1-Naphthyl, 2-Naphthyl, 3-Brom-2-naphthyl, 4-Methyl-1-naphthyl, 5-Methoxy-1-naphthyl, 6-Trifluormethyl-1-naphthyl, 7-Chlor-1-naphthyl, 8-Hydroxy-1-naphthyl;
   oder R⁴ bildet mit R⁵ zusammen mit dem benachbarten Kohlenstoffatom einen 3- bis 8-gliedrigen Ring;
- R⁵: Wasserstoff, C₁-C₄-Alkyl, C₃-C₆-Alkenyl, C₃-C₆-Alkinyl, C₁-C₄-Alkylcarbonyl, C₃-C₈-Cycloalkyl, C₁-C₄-Halogenalkyl, C₁-C₄-Alkoxyalkyl, C₁-C₄-Alkoxycarbonyl, C₁-C₄-Alkylthioalkyl oder Phenyl, oder R⁵ bildet mit R⁴ einen 3- bis 6-gliedrigen Ring wie oben angegeben;
- Y: Schwefel, Sauerstoff oder eine Einfachbindung;

Besonders bevorzugt sind Milchsäurederivate der Formel I, in der R⁵ für Methyl steht.

In der folgenden Tabelle sind beispielhaft einige der bevorzugten Verbindungen aufgeführt. Die darin für einen Substituenten genannten Gruppen stellen außerdem für sich betrachtet - unabhängig von der speziellen Kombination mit anderen Substituenten, in der sie genannt sind - eine besonders bevorzugte Definition des betreffenden Substituenten dar.

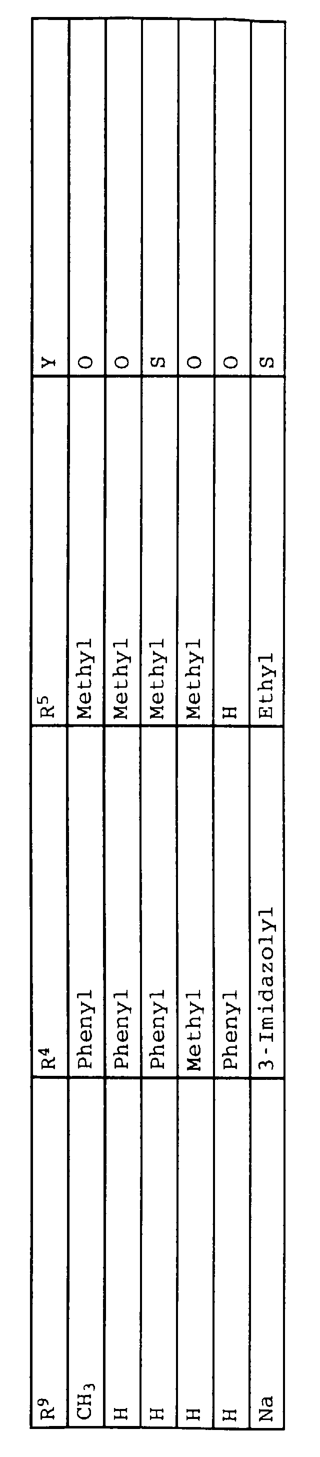

Die substituierten Milchsäurederivate I eignen sich auch als Antidots, um herbizide Wirkstoffe für Kulturpflanzen wie Kulturhirse, Reis, Mais, Getreidearten (Weizen, Roggen, Gerste, Hafer), Baumwolle, Zuckerrüben, Zuckerrohr und Soja verträglicher zu machen. Sie wirken antagonistisch auf Herbizide verschiedenster Stoffklassen wie Triazine, Phenylharnstoffderivate, Carbamate, Thiolcarbamate, Halogenacetanilide, Benzoesäurederivate sowie insbesondere Halogenphenoxyessigsäureester, substituierte Phenoxyphenoxyessigsäureester, Phenoxyphenoxypropionsäureester und Cyclohexenonderivate.

Herbizid wirksame Cyclohexenon-Derivate XIII sind beispielsweise aus EP-A 228 598, EP-A 230 235, EP-A 238 021, EP-A 368 227, US-A 4 432 786, DE-A 24 39 104, DE-A 38 38 309 und EP-A 537 463 bekannt. Sie dienen vorwiegend zur Bekämpfung unerwünschter Gräser in dicotylen Kulturen und in Gräsern, die nicht zur Familie der Gramineen zählen. In Abhängigkeit der Substituenten und der Dosierung der Verbindungen des Typs XIII bei ihrer Anwendung können diese Cyclohexenone auch zur selektiven Bekämpfung von unerwünschten Gräsern in Gramineen-Kulturen wie Weizen und Reis eingesetzt werden.

Beispielsweise haben die Substituenten in Formel XIII die in EP-A 537 463 genannte Bedeutung wie:
- R^{a}: eine C₁-C₆-Alkylgruppe;
- R^{b}: Wasserstoff, das Äquivalent eines landwirtschaftlich brauchbaren Kations, eine C₂-C₈-Alkylcarbonylgruppe, eine C₁-C₁₀-Alkylsulfonylgruppe, eine C₁-C₁₀-Alkylphosphonylgruppe oder die Benzoyl-, Benzolsulfonyl- oder Benzolphosphonylgruppe, wobei die drei letztgenannten Gruppen noch je 1 bis 5 Halogenatome tragen können;
- R^{c}: Wasserstoff, die Cyanogruppe, die Formylgruppe, eine C₁-C₆-Alkylgruppe, eine C₁-C₄-Alkoxy-C₁-C₆-alkyl- oder C₁-C₄-Alkylthio-C₁-C₆-alkylgruppe, eine Phenoxy-C₁-C₆-alkyl-, Phenylthio-C₁-C₆-alkyl-, Pyridyloxy-C₁-C₆-alkyl- oder Pyridylthio-C₁-C₆-alkylgruppe, wobei die Phenyl- und Pyridylringe jeweils noch einen bis drei Reste tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{g}R^{h},
wobei
- R^{g}: Wasserstoff, C₁-C₄-Alkyl, C₃-C₆-Alkenyl, C₃-C₆-Alkinyl, C₁-C₆-Acyl oder Benzoyl, das einen bis drei Reste tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio und
- R^{h}: Wasserstoff, C₁-C₄-Alkyl, C₃-C₆-Alkenyl oder C₃-C₆-Alkinyl
bedeuten;
eine C₃-C7-Cycloalkyl- oder eine C5-C7-Cycloalkenylgruppe, wobei diese Gruppen noch einen bis drei Reste tragen können, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio, Benzylthio, C₁-C₄-Alkylsulfonyl, C₁-C₄-Alkylsulfenyl und C₁-C₄-Alkylsulfinyl,
ein 5-gliedriger gesättigter Heterocyclus, der ein oder zwei Sauerstoff- oder Schwefelatome oder ein Sauerstoff- und ein Schwefelatom als Heteroatome enthält, und der noch einen bis drei Reste tragen kann, ausgewählt aus einer Gruppe bestehend aus C₁-C₄-Alkyl, partiell oder vollständig halogeniertem C₁-C₄-Alkyl, C₁-C₄-Alkoxy und C₁-C₄-Alkylthio,
ein 6- oder 7-gliedriger gesättigter oder ein- oder zweifach ungesättigter Heterocyclus, der ein oder zwei Sauerstoff- oder Schwefelatome oder ein Sauerstoff und ein Schwefelatom als Heteroatome enthält, und der noch einen bis drei Reste tragen kann, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio,
ein 5-gliedriger Heteroaromat, enthaltend ein bis drei Heteroatome, ausgewählt aus einer Gruppe bestehend aus ein oder zwei Stickstoffatomen und einem Sauerstoff- oder Schwefelatom, wobei der Heteroaromat noch einen bis drei Reste tragen kann, ausgewählt aus einer Gruppe bestehend aus Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₂-C₆-Alkenyl, C₂-C₆-Alkenyloxy, C₃-C₆-Alkinyloxy und C₁-C₄-Alkoxy-C₁-C₄-alkyl,
eine Phenyl- oder Pyridylgruppe, die jeweils noch einen bis drei Reste tragen kann, ausgewählt aus einer Gruppe bestehend aus Nitro, Cyano, Formyl, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{k}R^{l}, wobei R^{k} und R^{l} die oben genannte Bedeutung haben;
- R^{d}: Wasserstoff, die Hydroxylgruppe oder, wenn Rc für eine C₁-C₆-Alkylgruppe steht, eine C₁-C₆-Alkylgruppe;
- R^{e}: Wasserstoff, Halogen, die Cyanogruppe, eine C₁-C₄-Alkoxycarbonylgruppe oder eine C₁-C₄-Alkylketoximgruppe;
- W: eine C₁-C₆-Alkylen-, C₃-C₆-Alkenylen- oder C₃-C₆-Alkinylenkette, die jeweils noch ein bis drei Reste tragen können, ausgewählt aus einer Gruppe bestehend aus ein bis drei C₁-C₃-Alkylsubstituenten, ein bis drei Halogenatomen und einem Methylensubstituenten;
eine C₃-C₆-Alkylen- oder C₄-C₆-Alkenylenkette, die beide noch eine bis drei C₁-C₃-Alkylreste tragen können, wobei jeweils eine Methylengruppe der Ketten durch ein Sauerstoff- oder Schwefelatom, eine Sulfoxid- oder Sulfongruppe oder eine Gruppe -N(Rⁱ)- substituiert sein kann, wobei für Wasserstoff, C₁-C₄-Alkyl, C₃-C₆-Alkenyl oder C₃-C₆-Alkinyl steht;
- Rⁱ: Wasserstoff; die Vinylgruppe;
- R^{f}: eine Gruppe -CH=CH-Z, wobei Z für Cyano, Halogen, einen C₁-C₄-Alkylrest, einen partiell oder vollständig halogenierten C₁-C₄-Alkylrest, einen C₃-C₆-Cycloalkylrest, der seinerseits noch einen bis drei Substituenten tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy;
den Carboxylrest, einen C₁-C₈-Alkoxycarbonylrest, den Benzyloxycarbonylrest, den Phenyl-, Thienyl- oder Pyridylrest, wobei diese drei aromatischen Reste jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Cycloalkyl wobei der Cycloalkyl-Substituent seinerseits noch einen bis drei Reste tragen kann, ausgewählt aus einer Gruppe bestehend aus Halogen, C₁-C₄-Alkyl, partiell oder vollständig halogeniertem C₁-C₄-Alkyl und C₁-C₄-Alkoxy, steht;
die Ethinylgruppe, die einen der folgenden Reste tragen kann: einen C₁-C₄-Alkyl- oder C₃-C₆-Cycloalkylrest, die beide noch einen bis drei Substituenten tragen können, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy, oder den Phenyl-, Thienyl- oder Pyridylrest, wobei die aromatischen Reste jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy und C₁-C₄-Alkylthio;
die Phenylgruppe, eine Halogenphenylgruppe, eine Dihalogenphenylgruppe, eine 5-gliedrige heteroaromatische Gruppe mit ein bis drei Heteroatomen, ausgewählt aus einer Gruppe bestehend aus ein bis drei Stickstoffatomen und einem Sauerstoff- oder Schwefelatom, oder eine 6-gliedrige heteroaromatische Gruppe mit ein bis vier Stickstoffatomen, die nicht alle gleichzeitig benachbart sein können, wobei die Phenyl- und Heteroarylgruppen noch einen bis drei Reste tragen können, ausgewählt aus einer Gruppe bestehend aus Nitro, C₁-C₄-Alkoxyresten, C₁-C₄-Alkylthioresten, partiell oder vollständig halogenierten C₁-C₄-Alkoxyresten, Resten Z oder einem Rest -NR^{k}R^{l},
wobei
- R^{k}: Wasserstoff, C₁-C₄-Alkyl, C₃-C₆-Alkenyl oder C₃-C₆-Alkinyl und
- R^{l}: Wasserstoff, C₁-C₄-Alkyl, C₃-C₆-Alkenyl, C₃-C₆-Alkinyl, C₁-C₆-Acyl oder Benzoyl, das noch einen bis drei Substituenten tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio,
bedeuten.

Die Herstellung dieser Cyclohexenonderivate und ihre Anwendungsweise kann wie in EP-A-537 463 angegeben erfolgen.

Unter den Cyclohexenonoximethern der Formel XIII sind insbesondere die in EP-A 537 463, in den Tabellen 1 - 8 hervorgehobenen Verbindungen von besonderem Interesse. Im Hinblick auf ihre wirtschaftliche Bedeutung sind Mischungen der Milchsäurederivate I mit folgenden Cyclohexenonderivaten besonders bevorzugt:

| Nr. | Bezeichnung |
|---|---|
| XIII.1 | 2-(1-Ethoximino-butyl)-5-(2-ethylthiopropyl)-cyclohexan-1,3-dion [Handelsname: Poast®]; |
| XIII.2 | 2-[1-(trans-3-Chlorallyloximino)-butyl]-5-(2-ethylthiopropyl)-cyclohexan-1,3-dion [Handelsname: Select®]; |
| XIII.3 | 2-(1-Ethoximino-butyl)-5-mesityl-cyclohexan-1,3-dion [Handelsname: Grasp ]; |
| XIII.4 | 2-(1-Ethoximino-butyl)-5-tetrahydrothiopyran-3-yl-cyclohexan-1,3-dion [Handelsname: Focus^{®}]; |
| XIII.5 | 2-[1-(trans-3-Chlorallyloximino)-propyl]-5-tetrahydropyran-4-yl-cyclohexan-1,3-dion; |
| XIII.6 | 2-[1-(trans-3-Chlorallyloximino)-propyl]-5-(1-methylthiocyclopropyl)cyclohexan-1,3-dion; |
| XIII.7 | 2-{1-[4-(4-Chlorphenyl)-but-3-enyloximino]-propyl}-5-tetrahydrothiopyran-3-yl-cyclohexan-1,3-dion; |
| XIII.8 | 2-{1-[4-(4-Fluorphenyl)-but-3-enyloximino]-propyl}-5-tetrahydrothiopyran-3-yl-cyclo-hexan-1,3-dion; |
| XIII.9 | 2-{1-[2-(4-Chlorphenoxy)-propyloximino]-butyl}-5-tetrahydrothiopyran-3-yl-cyclohexan-1,3-dion. |

Die Verbindungen I bzw. die sie enthaltenden Mittel sowie deren umweltverträgliche Salze z.B. von Alkalimetallen und Erdalkalimetallen können als Herbizide in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon oder stark polare Lösungsmittel, wie N-Methylpyrrolidon oder Wasser in Betracht.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate i können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen zwischen 0,01 bis 95 Gew.-%, vorzugsweise zwischen 0,5 bis 90 Gew.-%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können weiterhin beispielsweise wie folgt formuliert werden:
I. 20 Gew.-Teile der Verbindung Nr. 1.2 werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
II. 20 Gew.-Teile der Verbindung Nr. 1.6 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenyl und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
III. 20 Gew.-Teile des Wirkstoffs Nr. 1.2 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinus besteht. Durch Eingießen und feines Verteilen der Lösung in 100 000 Gew.-Teilen Wasser erhält man eine wäßrige Dispersion, die 0,02 Gew.-% des Wirkstoffs enthält.
IV. 20 Gew.-Teile des Wirkstoffs Nr. 1.6 werden mit 3 Gew.-Teilen des Natriumsalzes der Diisobutylnaphthalin-α-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.
V. 3 Gew.-Teile des Wirkstoffs Nr. 1.2 werden mit 97 Gew.-Teilen feinteiligem Kaolin vermischt. Man erhält auf diese Weise ein Stäubemittel, das 3 Gew.-% des Wirkstoffs enthält.
VI. 20 Gew.-Teile des Wirkstoffs Nr. 1.6 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 10,0, vorzugsweise 0,01 bis 3,0 kg/ha aktive Substanz (a.S.).

In Anbetracht der Vielseitigkeit der Applikationsmethoden können die erfindungsgemäßen Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Unter Berücksichtigung der Vielseitigkeit der Applikationsmethoden können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicaco sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber lassen sich die Verbindungen I auch in Kulturen, die durch Züchtung und/oder mittels gentechnischer Methoden gegen die Wirkung von I oder anderen Herbiziden weitgehend resistent gemacht wurden, einsetzen.

Die Verbindungen der Formel I können weiterhin die verschiedenen Entwicklungsstadien einer Pflanze beeinflussen und deshalb als Wachstumsregulatoren eingesetzt werden. Die Wirkungsvielfalt der Pflanzenwachstumsregulatoren ist vor allem abhängig
a) von der Pflanzenart und -sorte,
b) von dem zeitpunkt der Applikation, bezogen auf das Entwicklungsstadium der Pflanze, und von der Jahreszeit,
c) von dem Applikationsort und -verfahren (z.B. Samenbeize, Bodenbehandlung, Blattapplikation oder Stamminjektion bei Bäumen),
d) von klimatischen Faktoren (z.B. Temperatur, Niederschlagsmenge, außerdem Tageslänge und Lichtintensität),
e) von der Bodenbeschaffenheit (einschließlich Düngung),
f) von der Formulierung bzw. Anwendungsform des Wirkstoffs und
g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt:
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern an Straßenrändern, Hekken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchte zu erhöhen, den Proteingehalt in Getreide oder Soja zu steigern oder Gummibäume zum vermehrten Latexfluß zu stimulieren.
   Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.
D. Durch den Einsatz der erfindungsgemäßen Substanzen laßt sich die Intensität der Bewässerung reduzieren und damit eine kostengünstigere Bewirtschaftung durchführen. Unter dem Einfluß von Wachstumsregulatoren kommt es nämlich zu einer besseren Ausnutzung des vorhandenen Wassers, weil u.a. die Öffnungsweite der Stomata reduziert wird, eine dickere Epidermis und Cuticula ausgebildet werden, die Durchwurzelung des Bodens verbessert wird und das Mikroklima im Pflanzenbestand durch einen kompakteren Wuchs günstig beeinflußt wird.

Besonders gut eignen sich die Verbindungen I zur Halmverkürzung von Kulturpflanzen wie Gerste, Raps und Weizen.

Die erfindungsgemäß zu verwendenden Wachstumsregulatoren der Formel I können den Kulturpflanzen sowohl von Samen her (als Saatgutbeizmittel) als auch über den Boden, d.h. durch die Wurzel sowie - besonders bevorzugt - durch Spritzung über das Blatt, zugeführt werden. Die Herstellung der Mittel erfolgt dabei analog zu der von Herbiziden (s.o.).

Die Aufwandmenge an Wirkstoff ist infolge der hohen Pflanzenverträglichkeit nicht kritisch. Die optimale Aufwandmenge variiert ja nach Bekämpfungsziel, Jahreszeit, Zielpflanzen und Wachstumsstadien.

Zur Verbreiterung des Wirkungsspektrums und zur Erzielung synergistischer Effekte können die Milchsäurederivate I mit zahlreichen Vertretern anderer herbizider oder wachstumsregulierender Wirkstoffgruppen gemischt und gemeinsam ausgebracht werden. Beispielsweise kommen als herbizide Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazoline, Sulfonamide, Sulfonylharnstoffe, Aryloxy-, Heteroaryloxyphenoxypropionsäuren sowie deren Salze, Ester und Amide und andere in Betracht. Bei Wachstumsregulatoren kommen insbesondere Chlormequatchlorid, Ethylen und Mepiquatchlorid in Frage.

Außerdem kann es von Nutzen sein, die Verbindungen I allein oder in Kombination mit anderen Herbiziden oder Wachstumsregulatoren auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die herbiziden Wirkstoffe XIII und die antidotisch wirkenden Verbindungen I können gemeinsam oder getrennt nach dem Auflaufen auf die Blätter und Sprosse der Kulturpflanzen und unerwünschten Pflanzen ausgebracht werden. Bevorzugt bringt man jedoch die herbiziden und antidotischen Wirkstoffe gleichzeitig auf das Feld. Bei getrennter Ausbringung von Antidot und herbizidem Wirkstoff wird vorzugsweise das Antidot zuerst ausgebracht.

Der antidotische und der herbizide Wirkstoff können gemeinsam oder getrennt formuliert werden und dann in suspendierender, emulgierbarer oder löslicher Form zur Bereitung von Spritzmitteln vorliegen.

Antidotische Effekte werden auch durch Behandlung der Kulturpflanzensamen oder der Stecklinge mit dem Antidot vor der Aussaat bzw. vor dem Auspflanzen erzielt. Der herbizide Wirkstoff wird dann allein in der üblichen Weise appliziert.

Bei der Saatgutbehandlung werden im allgemeinen Wirkstoffmengen von 0,01 bis 10 g, vorzugsweise 0,1 bis 2 g, je Kilogramm Saatgut benötigt.

Bei der Applikation des Antidots durch Samenquellung oder bei der Stecklingsbehandlung werden bevorzugt Lösungen eingesetzt, die den antagonistischen Wirkstoff in einer Konzentration von 1 bis 10 000 ppm, insbesondere von 100 bis 10 000 ppm, enthalten.

In den verschiedenen Pflanzenkulturen benötigt man üblicherweise unterschiedliche Mengen an antidotisch wirksamer Verbindung I und herbizider Verbindung XIII, wobei die Mengenverhältnisse in breiten Bereichen variabel sind. Sie sind abhängig von der Struktur der Cyclohexenon-Derivate, der substituierten Milchsäurederivate I und der jeweiligen Pflanzenkultur, auf die die Verbindungen ausgebracht werden. Geeignete Anteilsverhältnisse von herbizidem Wirkstoff zu antidotisch wirksamen substituierten Milchsäurederivaten I liegen zwischen 1:10 und 1:0,01, vorzugsweise zwischen 1:4 und 1:0,1.

Die Formulierungen enthalten 0,02 bis 95 Gew.-%, vorzugsweise 0,5 bis 90 Gew.-% an herbizidem Wirkstoff und Antidot. Die Aufwandmengen an herbizidem Wirkstoff betragen 0,05 bis 1 kg/ha.

### Synthesebeispiele

### Beispiel 1

### 3-Azido-3-phenyl-2-hydroxybuttersäuremethylester

19,5 g (300 mmol) Natriumazid und 16,0 g (300 mmol) Ammoniumchlorid werden in 400 ml Methanol suspendiert und mit 19,2 g (100 mmol) 3-Phenyl-2,3-epoxybuttersäuremethylester versetzt. Man rührt 9 Stunden unter Rückfluß und 12 Stunden bei Raumtemperatur, destilliert die Hauptmenge des Methanols ab und versetzt mit 200 ml Wasser. Dann wird mit Essigester extrahiert, die organischen Phasen getrocknet und eingeengt. Das verbleibende Öl wird mit wenig Diethylether versetzt und die gebildeten Kristalle abgesaugt.
Ausbeute: 6,3 g (27 %), Fp.: 110 - 112°C

### Beispiel 2

### 3-Azido-3-phenyl-2-(4,6-dimethoxypyrimidin-2-yl)oxybuttersäuremethylester

5,9 g (25 mmol) 3-Azido-3-phenyl-2-hydroxybuttersäuremethylester (Bsp. 1) werden in 80 ml DMF gelöst, mit 1,7 g (12,5 mmol) Kaliumcarbonat und 5,5 g (25 mmol) 4,6-Dimethoxy-2-methylsulfonylpyrimidin versetzt, 6 Stunden bei 50°C und 12 Stunden bei Raumtemperatur gerührt. Danach wird auf 400 ml Wasser gegossen, der gebildete Niederschlag abgesaugt, mit Wasser gewaschen und getrocknet. Man erhält 6,3 g eines weißen Pulvers.
Ausbeute: 67,2 %, Fp.: 118 - 120°C

### Beispiel 3

### 3-Azido-3-phenyl-2-(4,6-dimethoxypyrimidin-2-yl)oxybuttersäure

4,1 g (11 mmol) 3-Azido-3-phenyl-2-(4,6-dimethoxypyrimidin-2-yl)oxybuttersäure-methylester (Bsp. 2) werden in 100 ml Methanol/THF (1 : 1) gelöst und mit 13,2 g (33 mmol) 10 %iger Natronlauge versetzt. Man rührt 6 Stunden bei 50°C und 12 Stunden bei Raumtemperatur und engt am Rotationsverdampfer ein. Der Rückstand wird in 100 ml Wasser aufgenommen und mit 10 %iger Salzsäure auf pH 2 angesäuert. Der entstandene Niederschlag wird abgesaugt, mit Diethylether gewaschen und getrocknet.
Ausbeute: 1,5 g (26 %), Fp.: 193 - 195°C

### Beispiel 4

### 3-Azido-3-phenyl-2-[(4,6-dimethoxypyrimidin-2-yl)thio]buttersäuremethylester

5,9 g (25 mmol) 3-Azido-3-phenyl-2-hydroxybuttersäuremethylester (Bsp. 1) werden in 50 ml Dichlormethan gelöst, 3 g (30 mmol) Triethylamin zugegeben und unter Rühren 3,2 g (28 mmol) Methansulfonsäurechlorid zugetropft. Man rührt 23 Stunden bei Raumtemperatur, wäscht dreimal mit je 100 ml Wasser, trocknet über Natriumsulfat und engt im Vakuum ein. Der Rückstand wird in 100 ml DMF aufgenommen und bei 0°C zu einer Suspension von 12,9 g (75 mmol) 4,6-Dimethoxypyrimidin-2-thiol und 8,4 g (100 mmol) Natriumydrogencarbonat in 100 ml DMF getropft. Nach 2 Stunden Rühren bei Raumtemperatur und weiteren 2 Stunden bei 60°C gießt man auf 1 l Eiswasser und saugt den entstandenen Niederschlag ab.

Analog den obigen Beispielen wurden alle in Tabelle 1 genannten Verbindungen hergestellt.

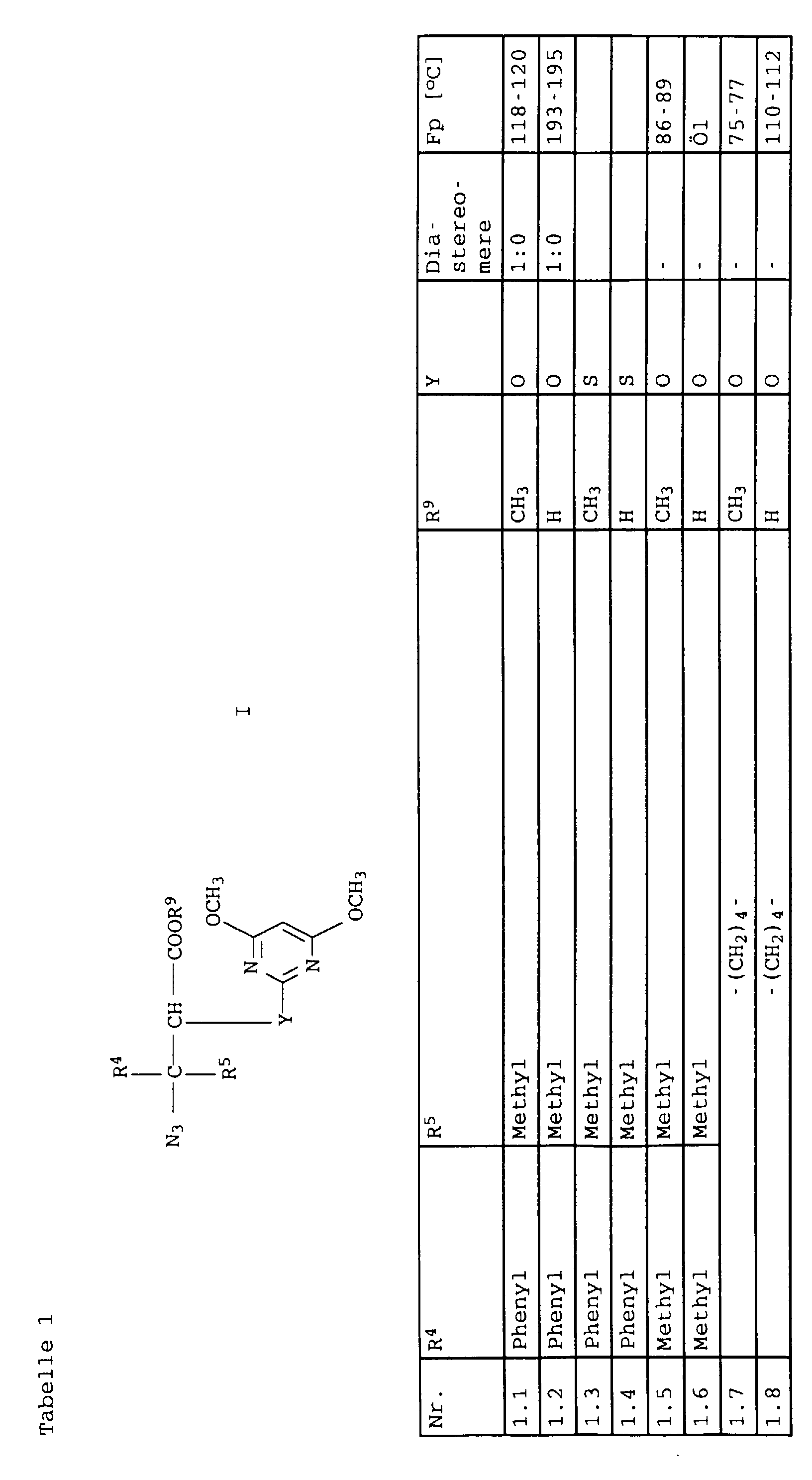

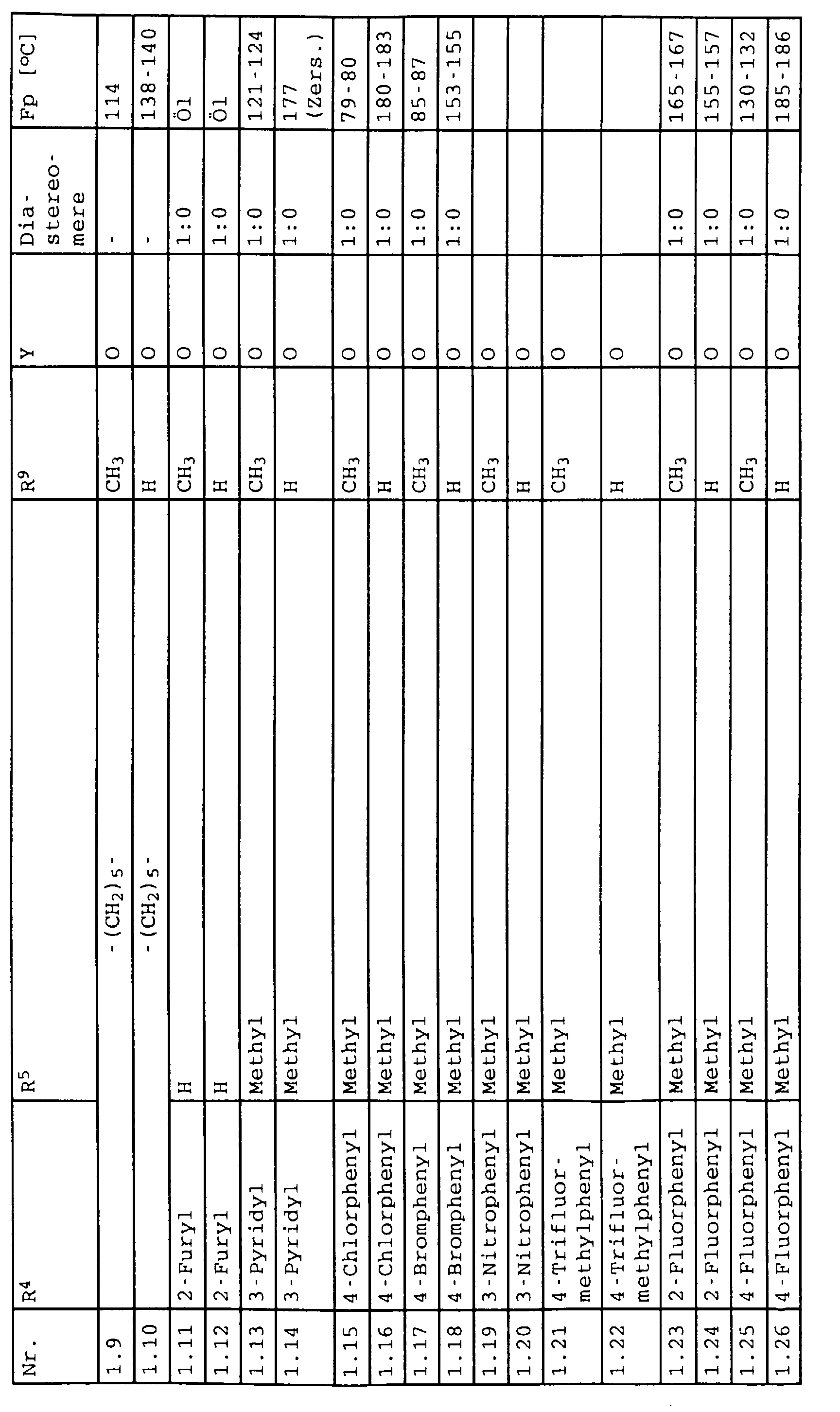

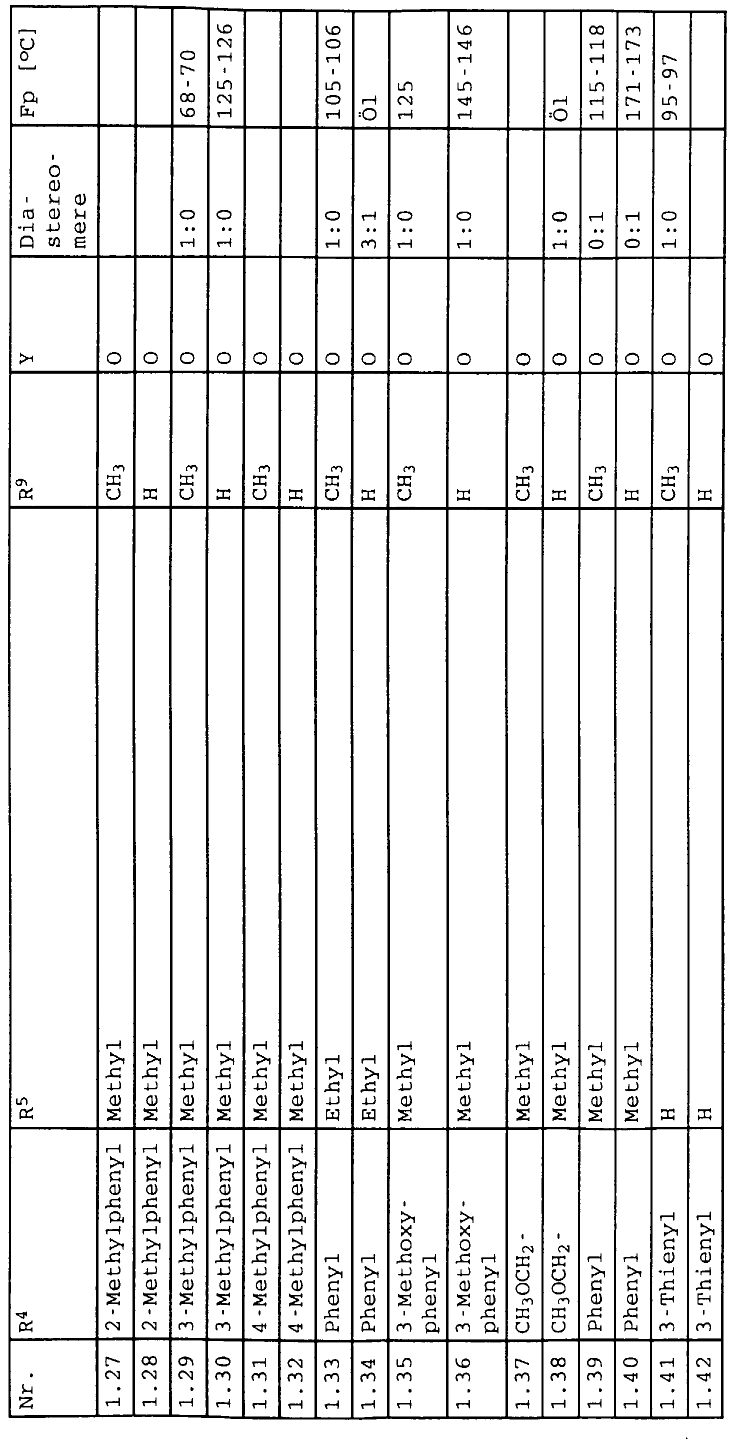

### Anwendungsbeispiele

Die herbizide und wachstumsregulatorische Wirkung der Milchsäurederivate der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufbehandlung betrug 3,0 kg/ha a.S.

Zum Zweck der Nachauflaufbehandlung wurden die Testpflanzen je nach Wuchsform erst bis zu einer Wuchshöhe 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 3,0 kg/ha a.S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Die herbizide Wirkung wurde nach einer Skala von 0 bis 100 bewertet. Dabei bedeutet 100 kein Aufgang der Pflanzen bzw. völlige Zerstörung zumindest der oberirdischen Teile und 0 keine Schädigung oder normaler Wachstumsverlauf.

Die wachstumsregulierende Wirkung wurde durch Längenmessung bestimmt. Bei Versuchsende wurden die Wuchshöhen der behandelten Pflanzen gemessen und zur Wuchshöhe von nicht behandelten Pflanzen in Beziehung gesetzt.

Die in den Gewächshausversuchen verwendeten Pflanzen setzten sich aus folgenden Arten zusammen:

| Lateinischer Name | Deutscher Name | Englischer Name |
|---|---|---|
| Echinochloa crusgalli | Hühnerhirse | barnyardgrass |
| Galium aparine | Klettenlabkraut | cleavers |
| Ipomoea ssp. | Prunkwinden-Arten | morningglory |
| Lolium multiflorum | Ital. Raygrass | ital. ryegrass |
| Sinapis alba | Weißer Senf | white mustard |
| Setaria italica | Ital. Borstenhirse | millet foxtail |
| Brassica napus | Raps | rape |
| Triticum aestivum | Weizen | wheat |
| Hordeum vulgare | Gerste | barley |

Die Ergebnisse zeigten eine sehr gute herbizide Wirkung der erfindungsgemäßen Verbindungen Nr. 1.2 und Nr. 1.6 im Vor- und Nachauflaufverfahren.

Der Einfluß verschiedener Vertreter der erfindungsgemäßen herbiziden Mittelkombinationen, bestehend aus Herbizid und antidotisch wirkender Verbindung, auf das Wachstum von erwünschten und unerwünschten Pflanzen im Vergleich zum herbiziden Wirkstoff allein wurde in Gewächshausversuchen belegt:

Bei Gewächshausversuchen dienten als Kulturpflanzen Plastiktöpfe mit rund 300 cm³ Inhalt und lehmigem Sand mit etwa 3,0 Gew.-% Humus als Substrat. Die Samen der Testpflanzen wurden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

Für die Nachauflaufbehandlung wurden die Testpflanzen je nach Wuchsform erst bis zu einer Wuchshöhe von 3 bis 20 cm angezogen und erst dann behandelt. Die herbiziden Mittel wurden hierbei in Wasser als Verteilungsmittel suspendiert oder emulgiert und mittels fein verteilender Düsen gespritzt.

Als Beispielherbizide der Cyclohexenon-Derivate XIII dienten 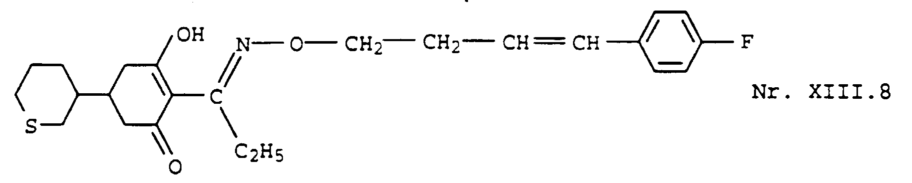 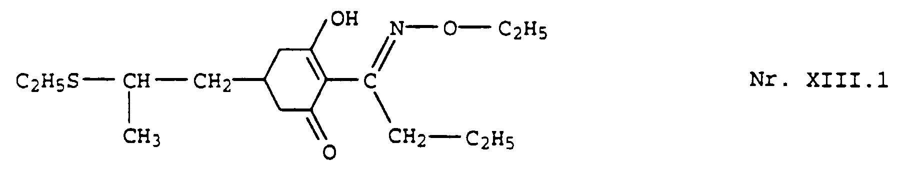 (Handelsname: Poast®, common name: Sethoxydim)

Sämtliche antidotisch wirkenden Verbindungen wurden für die Nachauflaufbehandlung in einem Gemisch, bestehend aus 80 Gew.-% Cyclohexanon als Verdünnungsmittel und 20 Gew.-% Emulphor® EL (ethoxyliertes Rizinusöl (caster-oil)) mit 10 Gew.-% Wirkstoff aufbereitet.

Zum Vergleich wurde der herbizide Wirkstoff als 10 bis 20 Gew.-% Emulsionskonzentrat formuliert und jeweils unter Zugabe von derjenigen Menge an Lösungsmittelsystem in die Spritzbrühe eingesetzt, mit welcher die antidotisch wirkende Verbindung in den Tabellen angegebenen Aufwandmengen ausgebracht wurden. Die Herstellung der Lösung erfolgte durch Einmischen des Wirkstoffs in eine Lösung auf 93 Gew.-% Xylol und 7 Gew.-% Lutensol® AP-8 (nichtionisches oberflächenaktives Mittel auf Basis von Alkylphenolpolyethylenglykolethern).

Nach Applikation der jeweiligen Wirkstoffmischung wurden die Testpflanzen im Gewächshaus kultiviert, und zwar wärmeliebende Arten bei etwa 18 bis 30°C, solche gemäßigterer Klimate bei ca. 10 bis 25°C.

Die Versuchsperiode erstreckte sich über 3 bis 5 Wochen. Während dieser Zeit wurden die Pflanzen gepflegt, wobei ihre Reaktionen auf die Wirkstoff-Behandlungen erfaßt wurden.

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Durch Verwendung der Milchsäurederivate I wurde die Verträglichkeit von herbiziden Cyclohexenon-Derivaten XIII für Kulturpflanzen aus der Familie der Gramineen (Gräser) wie Weizen und Mais deutlich verbessert.

## Patentansprüche

1. Substituierte Milchsäurederivate mit einem Rest in β-Position der allgemeinen Formel I 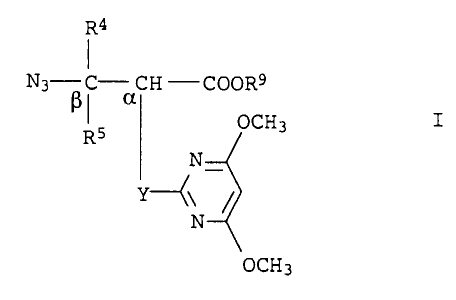 in der die Variablen folgende Bedeutung haben:
R⁹ Wasserstoff, ein Alkalimetallkation, das Äquivalent eines Erdalkalimetallkations, das Ammoniumkation oder ein organisches Ammoniumion;
C₃-C₈-Cycloalkyl, welches ein bis drei C₁-C₄-Alkylgruppen tragen kann; C₁-C₈-Alkyl, welches Halogen, C₁-C₄-Alkoxy, C₁-C₄-Alkylthio, Cyano, C₁-C₄-Alkylcarbonyl, C₃-C₈-Cycloalkyl, C₁-C₄-Alkoxycarbonyl, Phenyl oder durch Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio substituiertes Phenyl oder Phenoxy tragen kann;
C₃-C₆-Alkenyl oder C₃-C₆-Alkinyl, welche ein bis fünf Halogenatome tragen können;
Phenyl, welches Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio tragen kann;
R⁹ ferner eine Gruppe 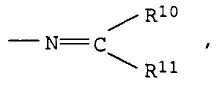 worin R¹⁰ und R¹¹, die gleich oder verschieden sein können, bedeuten:
C₁-C₈-Alkyl, C₃-C₆-Alkenyl, C₃-C₆-Alkinyl, C₃-C₈-Cycloalkyl, wobei diese Reste einen C₁-C₄-Alkoxy, C₁-C₄-Alkylthio oder einen Phenylrest tragen können, welcher Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio tragen kann;oder
Phenyl, welches Halogen, Nitro, Cyano, C₁-C₄-Alkyl, C₁-C₄-Halogenalkyl, C₁-C₄-Alkoxy, C₁-C₄-Halogenalkoxy und/oder C₁-C₄-Alkylthio tragen kann;
oder R¹⁰ und R¹¹ bilden gemeinsam eine C₃-C₁₂-Alkylenkette, welche ein bis drei C₁-C₄-Alkylgruppen tragen kann;
R⁴ C₃-C₈-Cycloalkyl, C₃-C₈-Cycloalkenyl, wobei diese Reste durch Halogen, C₁-C₄-Alkyl, C₁-C₄-Alkoxy, C₁-C₄-Alkylthio, Cyano, C₁-C₄-Alkylcarbonyl, C₁-C₄-Alkoxycarbonyl oder Phenyl substituiert sein können;
C₁-C₈-Alkyl, welches Halogen, C₁-C₄-Alkoxy, C₁-C₄-Alkylthio, Cyano, Hydroxy, C₁-C₄-Alkylcarbonyl, C₁-C₄-Alkoxycarbonyl, Phenyl, Phenoxy und/oder Phenylcarbonyl tragen kann;
C₃-C₆-Alkenyl oder C₃-C₆-Alkinyl, welche Halogen, C₁-C₄-Alkyl, C₁-C₄-Alkoxy, C₁-C₄-Alkylthio, Cyano, C₁-C₄-Alkylcarbonyl, C₁-C₄-Alkoxycarbonyl oder Phenyl tragen können;
Phenyl oder Naphthyl, welche Halogen, Nitro, Cyano, Hydroxy, Mercapto, C₁-C₄-Alkyl, C₁-C₄-Halogenalkyl, C₁-C₄-Alkoxy, C₁-C₄-Halogenalkoxy, C₁-C₄-Alkylthio, Amino, C₁-C₄-Alkylamino, C₁-C₄-Dialkylamino, C₁-C₄-Alkylcarbonyl oder C₁-C₄-Alkoxycarbonyl tragen können; oder
ein fünf- oder sechsgliedriger Heteroaromat, enthaltend ein bis drei Stickstoffatome und/oder ein Schwefel- oder Sauerstoffatom;
oder R⁴ und R⁵ bilden zusammen mit dem benachbarten Kohlenstoffatom einen 3- bis 8-gliedrigen Ring;
R⁵ Wasserstoff, C₁-C₄-Alkyl, C₃-C₆-Alkenyl; C₃-C₆-Alkinyl, C₁-C₄-Alkylcarbonyl, C₁-C₄-Alkoxycarbonyl, C₁-C₄-Halogenalkyl, C₃-C₈-Cycloalkyl, C₁-C₄-Alkoxyalkyl, C₁-C₄-Alkylthioalkyl oder Phenyl,
oder R⁵ ist mit R⁴ wie oben angegeben zu einem Ring verknüpft;
Y Schwefel oder Sauerstoff.

2. Milchsäurederivate der Formel I gemäß Anspruch 1, in der R⁵ für Methyl steht.

3. Milchsäurederivate der Formel I gemäß Anspruch 1, wobei
a) R⁴ Phenyl, R⁵ Methyl, R⁹ Wasserstoff und Y Sauerstoff,
b) R⁴ Methyl, R⁵ Methyl, R⁹ Wasserstoff und Y Sauerstoff,
c) R⁴ 2-Fluorphenyl, R⁵ Methyl, R⁹ Wasserstoff und Y Sauerstoff,
d) R⁴ Phenyl, R⁵ Ethyl, R⁹ Wasserstoff und Y Sauerstoff,
e) R⁴ 3-Thienyl, R⁵ Wasserstoff, R⁹ Methyl und Y Sauerstoff,
f) R⁴ 3-Thienyl, R⁵ Wasserstoff, R⁹ Wasserstoff und Y Sauerstoff
bedeutet.

4. Herbizides Mittel, enthaltend eine Verbindung der Formel I gemäß den Ansprüchen 1 - 3 und übliche inerte Zusatzstoffe.

5. Mittel zur Regulierung des Pflanzenwachstums, enthaltend eine Verbindung der Formel I gemäß den Ansprüchen 1 - 3 und übliche inerte Zusatzstoffe.

6. Verfahren zur Bekämpfung unerwünschten Pflanzenwuchses, dadurch gekennzeichnet, daß man eine herbizid wirksame Menge einer Verbindung der Formel I gemäß den Ansprüchen 1 - 3 auf die Pflanzen oder deren Lebensraum einwirken läßt.

7. Verfahren zur Regulierung des Pflanzenwachstums, dadurch gekennzeichnet, daß man eine bioregulatorisch wirksame Menge einer Verbindung der Formel I gemäß den Ansprüchen 1 - 3 auf die Pflanzen oder deren Lebensraum einwirken läßt.

## Claims

1. A substituted lactic acid derivative having a radical in the β-position, of the general formula I 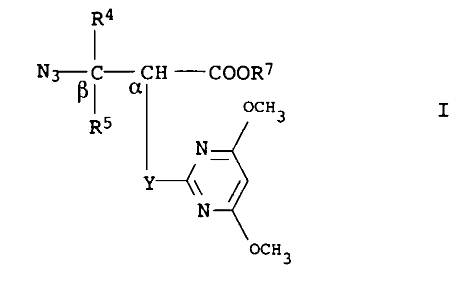 in which the variables have the following meanings:
R⁹ is hydrogen, an alkali metal cation, the equivalent of an alkaline earth metal cation, the ammonium cation, or an organic ammonium ion;
C₃-C₈-cycloalkyl, which can carry one to three C₁-C₄-alkyl groups; C₁-C₈-alkyl which can carry halogen, C₁-C₄-alkoxy, C₁-C₄-alkylthio, cyano, C₁-C₄-alkylcarbonyl, C₃-C₈-cycloalkyl, C₁-C₄-alkoxycarbonyl, phenyl, or phenyl or phenoxy substituted by halogen, nitro, cyano, C₁-C₄-alkyl, C₁-C₄-haloalkyl, C₁-C₄-alkoxy, C₁-C₄-haloalkoxy and/or C₁-C₄-alkylthio;
C₃-C₆-alkenyl or C₃-C₆-alkynyl which can carry one to five halogen atoms;
phenyl which can carry halogen, nitro, cyano, C₁-C₄-alkyl, C₁-C₄-haloalkyl, C₁-C₄-alkoxy, C₁-C₄-haloalkoxy and/or C₁-C₄-alkylthio;
R⁹ is furthermore a group 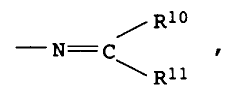 in which R¹⁰ and R¹¹, which can be identical or different, are:
C₁-C₈-alkyl, C₃-C₆-alkenyl, C₃-C₆-alkynyl, C₃-C₈-cycloalkyl, where these radicals can carry a C₁-C₄-alkoxy, C₁-C₄-alkylthio or a phenyl radical which can carry halogen, nitro, cyano, C₁-C₄-alkyl, C₁-C₄-haloalkyl, C₁-C₄-alkoxy, C₁-C₄-haloalkoxy and/or C₁-C₄-alkylthio; or
phenyl which can carry halogen, nitro, cyano, C₁-C₄-alkyl, C₁-C₄-haloalkyl, C₁-C₄-alkoxy, C₁-C₄-haloalkoxy and/or C₁-C₄-alkylthio;
or R¹⁰ and R¹¹ together form a C₃-C₁₂-alkylene chain which can carry one to three C₁-C₄-alkyl groups;
R⁴ is C₃-C₈-cycloalkyl, C₃-C₈-cycloalkenyl, where these radicals can be substituted by halogen, C₁-C₄-alkyl, C₁-C₄-alkoxy, C₁-C₄-alkylthio, cyano, C₁-C₄-alkylcarbonyl, C₁-C₄-alkoxycarbonyl or phenyl;
C₁-C₈-alkyl which can carry halogen, C₁-C₄-alkoxy, C₁-C₄-alkylthio, cyano, hydroxy, C₁-C₄-alkylcarbonyl, C₁-C₄-alkoxycarbonyl, phenyl, phenoxy and/or phenylcarbonyl;
C₃-C₆-alkenyl or C₃-C₆-alkynyl which can carry halogen, C₁-C₄-alkyl, C₁-C₄-alkoxy, C₁-C₄-alkylthio, cyano, C₁-C₄-alkylcarbonyl, C₁-C₄-alkoxycarbonyl or phenyl;
phenyl or naphthyl which can carry halogen, nitro, cyano, hydroxy, mercapto, C₁-C₄-alkyl, C₁-C₄-haloalkyl, C₁-C₄-alkoxy, C₁-C₄-haloalkoxy, C₁-C₄-alkylthio, amino, C₁-C₄-alkylamino, C₁-C₄-dialkylamino, C₁-C₄-alkylcarbonyl or C₁-C₄-alkoxycarbonyl; or
a five- or six-membered heteroaromatic, containing one to three nitrogen atoms and/or a sulfur or oxygen atom;
or R⁴ and R⁵, together with the adjacent carbon atom, form a 3- to 8-membered ring;
R⁵ is hydrogen, C₁-C₄-alkyl, C₃-C₆-alkenyl, C₃-C₆-alkynyl, C₁-C₄-alkylcarbonyl, C₁-C₄-alkoxycarbonyl, C₁-C₄-haloalkyl, C₃-C₈-cycloalkyl, C₁-C₄-alkoxyalkyl, C₁-C₄-alkylthioalkyl or phenyl,
or R⁵ is linked to R⁴ as indicated above to give a ring;
Y is sulfur or oxygen.

2. A lactic acid derivative of the formula I as claimed in claim 1, in which R⁵ is methyl.

3. A lactic acid derivative of the formula I as claimed in claim 1, where
a) R⁴ is phenyl, R⁵ is methyl, R⁹ is hydrogen and Y is oxygen,
b) R⁴ is methyl, R⁵ is methyl, R⁹ is hydrogen and Y is oxygen,
c) R⁴ is 2-fluorophenyl, R⁵ is methyl, R⁹ is hydrogen and Y is oxygen,
d) R⁴ is phenyl, R⁵ is ethyl, R⁹ is hydrogen and Y is oxygen,
e) R⁴ is 3-thienyl, R⁵ is hydrogen, R⁹ is methyl and Y is oxygen,
f) R⁴ is 3-thienyl, R⁵ is hydrogen, R⁹ is hydrogen and Y is oxygen.

4. A herbicidal composition containing a compound of the formula I as claimed in claims 1 - 3 and customary inert additives.

5. A composition for regulating plant growth, containing a compound of the formula I as claimed in claims 1 - 3 and customary inert additives.

6. A method of controlling undesired plant growth, which comprises allowing a herbicidally active amount of a compound of the formula I as claimed in claims 1 - 3 to act on the plants or their habitat.

7. A method for regulating plant growth, which comprises allowing an amount of a compound of the formula I as claimed in claims 1 - 3 which has bioregulatory action to act on the plants or their habitat.

## Revendications

1. Dérivés substitués de l'acide lactique portant un radical en position bêta et répondant à la formule générale I 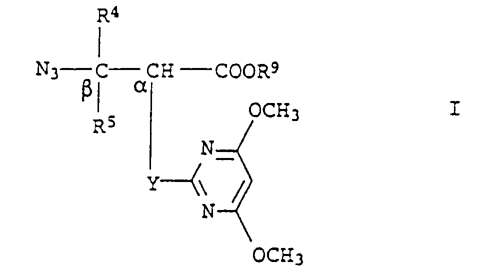 dans laquelle les symboles ont les significations suivantes :
R⁹ l'hydrogène, un cation de métal alcalin, un équivalent d'un cation de métal alcalino-terreux, le cation ammonium ou un ion ammonium organique;
un groupe cycloalkyle en C3-C8 qui peut porter un à trois groupes alkyle en C1-C4 ;
un groupe alkyle en C1-C8 qui peut porter des substituants halogéno, alcoxy en C1-C4, alkylthio en C1-C4, cyano, (alkyle en C1-C4)carbonyle, cycloalkyle en C3-C8, (alcoxy en C1-C4)carbonyle, phényle ou des substituants phényle ou phénoxy portant eux-mêmes des substituants halogéno, nitro, cyano, alkyle en C1-C4, halogénoalkyle en C1-C4, alcoxy en C1-C4, halogénoalcoxy en C1-C4 et/ou alkylthio en C1-C4 ;
un groupe alcényle en C3-C6 ou alcynyle en C3-C6 qui peut porter un à cinq atomes d'halogènes ;
un groupe phényle qui peut porter des substituants halogéno, nitro, cyano, alkyle en C1-C4, halogénoalkyle en C1-C4, alcoxy en C1-C4, halogénoalcoxy en C1-C4 et/ou alkylthio en C1-C4 ;
R⁹ peut en outre représenter un groupe 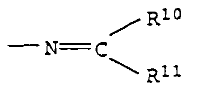 dans lequel R¹⁰ et R¹¹, qui peuvent être identiques ou différents, représentent :
des groupes alkyle en C1-C8, alcényle en C3-C6, alcynyle en C3-C6, cycloalkyle en C3-C8, lesquels peuvent porter un substituant alcoxy en C1-C4, alkylthio en C1-C4 ou un groupe phényle lequel peut porter des substituants halogéno, nitro, cyano, alkyle en C1-C4, halogénoalkyle en C1-C4, alcoxy en C1-C4, halogénoalcoxy en C1-C4 et/ou alkylthio en C1-C4 ; ou bien
des groupes phényle qui peuvent porter des substituants halogéno, nitro, cyano, alkyle en C1-C4, halogénoalkyle en C1-C4, alcoxy en C1-C4, halogénoalcoxy en C1-C4 et/ou alkylthio en C1-C4 ;
ou bien R¹⁰ et R¹¹ forment ensemble une chaîne alkylène en C3-C12 qui peut porter un à trois groupes alkyle en C1-C4 ;
R⁴ représente un groupe cycloalkyle en C3-C8, cycloalcényle en C3-C8, ces groupes pouvant eux-mêmes être substitués par des halogènes, des groupes alkyle en C1-C4, alcoxy en C1-C4, alkylthio en C1-C4, cyano, (alkyle en C1-C4)carbonyle, (alcoxy en C1-C4)carbonyle ou phényle ;
un groupe alkyle en C1-C8 qui peut porter des substituants halogéno, alcoxy en C1-C4, alkylthio en C1-C4, cyano, hydroxy, (alkyle en C1-C4)carbonyle, (alcoxy en C1-C4)carbonyle, phényle, phénoxy ou phénylcarbonyle;
un groupe alcényle en C3-C6 ou alcynyle en C3-C6 qui peut porter des substituants halogéno, alkyle en C1-C4, alcoxy en C1-C4, alkylthio en C1-C4, cyano, (alkyle en C1-C4)carbonyle, (alcoxy en C1-C4)carbonyle ou phényle ;
un groupe phényle ou naphtyle qui peut porter des substituants halogéno, nitro, cyano, hydroxy, mercapto, alkyle en C1-C4, halogénoalkyle en C1-C4, alcoxy en C1-C4, halogénoalcoxy en C1-C4, alkylthio en C1-C4, amino, alkylamino en C1-C4, di-(alkyle en C1-C4)amino, (alkyle en Cl-C4)carbonyle ou (alcoxy en C1-C4)carbonyle ; ou bien
un groupe hétéroaromatique à cinq à six chaînons contenant un à trois atomes d'azote et/ou un atome de soufre ou d'oxygène ;
ou bien R⁴ et R⁵ forment ensemble et avec l'atome de carbone voisin, un cycle de trois à huit chaînons ;
R⁵ représente l'hydrogène, un groupe alkyle en C1-C4, alcényle en C3-C6, alcynyle en C3-C6, (alkyle en C1-C4)carbonyle, (alcoxy en C1-C4)carbonyle, halogénoalkyle en C1-C4, cycloalkyle en C3-C8, (alcoxy en C1-C4)alkyle, (alkyle en C1-C4)thioalkyle ou phényle, ou bien R⁵ est relié avec R⁴, tel que défini ci-dessus, avec formation d'un cycle ;
Y représente le soufre ou l'oxygène.

2. Dérivés de l'acide lactique de formule I de la revendication 1, dans laquelle R⁵ représente un groupe méthyle.

3. Dérivés de l'acide lactique de formule I de la revendication 1, dans laquelle
a) représente un groupe phényle, R⁵ un groupe méthyle, R⁹ l'hydrogène et Y l'oxygène,
b) R⁴ représente un groupe méthyle, R⁵ un groupe méthyle, R⁹ l'hydrogène et Y l'oxygène,
c) R⁴ représente un groupe 2-fluorophényle, R⁵ un groupe méthyle, R⁹ l'hydrogène et Y l'oxygène,
d) R⁴ représente un groupe phényle, R⁵ un groupe éthyle, R⁹ l'hydrogène et Y l'oxygène,
e) R⁴ représente un groupe 3-thiényle, R⁵ l'hydrogène, R⁹ un groupe méthyle et Y l'oxygène,
f) R⁴ représente un groupe 3-thiényle, R⁵ l'hydrogène, R⁹ l'hydrogène, Y l'oxygène.

4. Produit herbicide contenant un composé de formule I selon les revendications 1 à 3 et des additifs inertes usuels.

5. Produit pour la régulation de la croissance des végétaux, contenant un composé de formule I selon les revendications 1 à 3 et des additifs inertes usuels.

6. Procédé pour combattre les croissances de végétaux indésirables, caractérisé par le fait que l'on fait agir une quantité herbicide efficace d'un composé de formule I selon les revendications 1 à 3 sur les végétaux ou leur habitat.

7. Procédé pour la régulation de la croissance des végétaux, caractérisé par le fait que l'on fait agir une quantité biorégulatrice efficace d'un composé de formule I selon les revendications 1 à 3 sur les végétaux ou leur habitat.
